Europäisches Patentamt

European Patent Office

Office européen des brevets

(19)

(11) Veröffentlichungsnummer: **0 027 829**
**B1**

(12) **EUROPÄISCHE PATENTSCHRIFT**

(45) Veröffentlichungstag der Patentschrift: **29.06.83**

(21) Anmeldenummer: **79104156.9**

(22) Anmeldetag: **26.10.79**

(51) Int. Cl.³: **C 12 P 33/06, C 07 J 1/00**

(54) Verfahren zur Herstellung von 9-Alpha-hydroxy-4-androsten-3,17-dion.

(43) Veröffentlichungstag der Anmeldung:
**06.05.81 Patentblatt 81/18**

(45) Bekanntmachung des Hinweises auf die Patenterteilung:
**29.06.83 Patentblatt 83/26**

(84) Benannte Vertragsstaaten:
**AT BE CH DE FR GB IT NL**

(56) Entgegenhaltungen:
**FR - A - 2 328 718**
**US - A - 3 080 298**
**US - A - 3 116 220**

(73) Patentinhaber: **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder: **Kieslich, Klaus, Dr.**
**Strasse zum Löwen 4**
**D-1000 Berlin 39 (DE)**

Courier Press, Leamington Spa, England.

Verfahren zu Herstellung von 9-Alpha-hydroxy- 4-androsten-3,17-dion

Die Erfindung betrifft das im Patentanspruch gekennzeichnete Verfahren.

Das 9α-Hydroxy-4-androsten-3,17-dion ist eine seit langem bekannte Substanz (J. Amer. Chem. Soc., *80*, 1958, 6148), welche sich durch eine antiandrogene und antiöstrogene Wirksamkeit anszeichnet und welche darüberhinaus ein wertvolles Zwischenprodukt zur Herstellung von 4,9(11)-Androstadien-3,17-dion ist, das seinerseits mittels bekannter Methoden in zahlreiche pharmakologisch wirksame Steroide überführt werden kann.

Bei der mikrobiologischen Herstellung dieser Verbindung aus 4-Androsten-3,17-dion nach den bisher bekannten Verfahren kann man nur relativ geringe Ausbeuten an Verfahrensprodukt erzielen (Biotechnology and Bioenginee ring XI, 1969, 1183; US—Patente 3,080,298 und 3,116,200).

Demgegenüber ist es mit Hilfe des erfindungsgemäßen Verfahrens möglich, hohe Substratkonzentrationen von 4-Androsten-3,17-dion mit hoher Ausbeute in 9α-Hydroxy-4-androsten-3,17-dion zu überführen.

Das erfindungsgemäße Verfahren wird unter den Bedingungen durchgeführt, die man üblicherweise bei der mikrobiologischen Hydroxylierung von Steroiden anwendet.

Unter den üblicherweise verwendeten Kulturbedingungen werden in einem geeigneten Nährmedium unter Belüften, Submerskulturen angezüchtet. Dann setzt man den Kulturen das 4-Androsten-3,17-dion (in einem Lösungsmittel gelöst oder vorzugsweise in emulgierter Form) zu und fermentiert, bis eine maximale Substratumwandlung erreicht ist.

Geeignete Substratlösungsmittel sind beispielsweise Methanol, Äthanol, Glykolmonomethyläther, Dimethylformamid oder Dimethylsulfoxyd. Die Emulgierung des Substrats kann beispielsweise bewirkt werden, indem man dieses in mikronisierter Form oder in einem mit Wasser mischbaren Lösungsmittel (wie Methanol, Äthanol, Aceton, Glykolmonomethyläther, Dimethylformamid oder Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Äthylenoxyaddukte oder Fettsäureester von Polyglykolen. Als geeignete Emulgatoren seien die handelsüblichen Netzmittel Tegin[R], Tween[R], und Span[R] beispielsmäßig genannt.

Die optimale Substratkonzentration, Substratzugabezeit und Fermentationsdauer ist von den angewendeten Fermentationsbedingungen abhängig. Diese Größen müssen, wie dies bei mikrobiologischen Steroidumwandlungen allgemein erforderlich ist, im Einzelfall durch Vorversuche, wie sie dem Fachmann geläufig sind, ermittelt werden.

Die nachfolgenden Beispiele dienen zur Erläuterung des erfindungsgemäßen Verfahrens.

Beispiel 1

500 ml eines sterilisierten Mediums in einem 2 l Erlenmeyerkolben bestehend aus 6% flüssigem Stärkezucker (entsprechend 3% fester Glucose)

    1% Cornsteep liquor
    0,2% Natriumnitrat
    0,1% Kaliumdihydrogenphosphat
    0,2% Dikaliumhydrogenphosphat
    0,05% Magnesiumsulfat
    0,002% Eisen(II)-sulfat
    0,05% Kaliumchlorid

werden beimpft mit einer Abschwemmung einer ein- bis zwei Wochen alten Schrägagarkultur von Corynespora cassicola ATCC 16718. Nach dreitägigem Wachstum auf einem Rotationsschüttler bei 30°C wird die Mycelsuspension als Inoculum für die Beimpfung eines 30 l Versuchsfermenters verwendet, der mit 25 l eines sterilisierten Mediums der gleichen Zusammensetzung beschickt ist. Die Germination wird bei 29°C, einer Belüftung von 25 l pro Minute und einer Rührgeschwindigkeit von 220 U/Min. durchgeführt und unter gelegentlicher Zugabe von Polyol 2000 als Antischaummittel. Nach 48 Stunden werden 1,5 l der Kultur unter sterilen Bedingungen in einen gleichgroßen Fermenter überführt, der mit 23,5 l einer sterilen Nährlösung der gleichen Zusammensetzung beschickt ist, und unter gleichen technischen Bedingungen betrieben. Nach 6 Stunden wird eine sterilfiltrierte Lösung von 12,5 g 4-Androsten-3,17-dion in 100 ml Dimethylformamid zugesetzt. Diese Substratzugabe wird bei 15 Stunden und 20 Stunden wiederholt, sodaß eine Substratkonzentration von 1,5 g/l erreicht wird. Die Umwandlung wird durch Entnahme mehrerer Proben kontrolliert, die mit Methylisobutylketon extrahiert und durch Dünnschichtchromatographie an Kieselgelplatten Merck 60 F-254 analysiert werden (Entwicklung Chloroform/Methanol 9+1). Nach ca. 38 Stunden Gesamtfermentationszeit ist das Ausgangsmaterial vollständig umgewandelt.

Der Fermenter wird geerntet, indem man das Mycel abschleudert und dreimal mit Methylisobutylketon extrahiert. Das Mycel wird ebenfalls mit Methylisobutylketon ausgewaschen. Die vereinigten Extrakte und Waschlösungen werden im Vakuum eingeengt, wobei bereits reine Produktmengen auskristallisieren und abfiltriert werden. 13,1 g (Schmelzpunkt 217/219—221°C).

Das Extraktkonzentrat wird zur Trockne eingeengt, der Rückstand mit Hexan von Antischaummittel freigewaschen und aus Essigester umkristallisiert. Es werden als Erst- und Zweitkristallisate nochmals 14,4 g (Schmelzpunkt 217/218—220°C) erhalten. Nach spektralen Daten besteht das Produkt einheitlich aus 9α-Hydroxy-4-androsten-3,17-dion. Die Gesamtausbeute beträgt 69,5% der Theorie.

Beispiel 2

Unter gleichen Bedingungen wie in Beispiel 1 beschrieben, werden 13 l gut entwickelter Kultur von Corynespora cassicola ATCC 16718 hergestellt, die bei 15 Stunden, 20 Stunden, 25 Stunden und 30 Stunden mit je 500 ml einer Substratsuspension beschickt werden. Die Substratsuspension wird hergestellt durch 2-stündiges Vermahlen von je 37,5 g in je 500 ml 2 %iger Tween(R)80-lösung in einer Kugelmühle. Die Substratsuspension wird bei 100°C pasteurisiert.

Nach 92 Stunden Gesamtfermentationszeit ist das Substrat umgewandelt. Es wird wie in Beispiel 1 beschrieben geerntet und aufgearbeitet. Beim Einengen der Methylisobutylketon-Extrakte werden 30 g Rohprodukt erhalten, was aus Essigester umkristallisiert wird und 25 g Reinprodukt ergibt. Das durch Auswaschen des Mycels erhaltene Rohprodukt von 50 g liefert aus Essigester umkristallisiert weitere 41 g Reinprodukt. Schmelzpunkt 218/219—221°C. Gesamtausbeute 70% der Theorie. Durch chromatographische Aufreinigung der Mutterlaugen sind weitere Produktmengen gewinnbar.

**Patentansprüch**

Verfahren zur Herstellung von 9α-Hydroxy-4-androsten-3,17-dion, dadurch gekennzeichnet, daß man 4-Androsten-3,17-dion mit einer Kultur von Corynespora cassicola ATCC 16 718 fermentiert.

**Revendication**

Procédé de fabrication de l'hydroxy-9α androstène-4 dione-3,17 caractérisé en ce qu'on fait fermenter l'androstène-4-dione-3,17 avec une culture de Corynespora cassicola ATCC 16 718.

**Claim**

Process for the manufacture of 9α-hydroxy-4-androstene-3,17-dione, characterised in that 4-androstene-3,17-dione is fermented with a culture of *Corynespora cassicola* ATCC 16 718.